# EUROPEAN PATENT SPECIFICATION

(11) **EP 2 833 914 B1**
(45) Date of publication and mention of the grant of the patent: **16.01.2019**
(21) Application number: 13772028.0
(22) Date of filing: 03.04.2013
(51) Int. Cl.: A61K 39/39, A61K 38/38, A61K 31/70, A61K 9/14, A61P 37/00

(54) **ADJUVANT SYSTEM FOR ORAL VACCINE ADMINISTRATION**
ADJUVANSSYSTEM ZUR ORALEN IMPFSTOFFVERABREICHUNG
SYSTÈME ADJUVANT POUR VACCIN ADMINISTRÉ PAR VOIE ORALE

(30) Priority: 04.04.2012 US 201261686372 P
(43) Date of publication of application: 11.02.2015
(73) Proprietor: Vaxform LLC, Lehigh Valley, PA (US)
(72) Inventor: MOREFIELD, Garry, Lehigh Valley, PA (US)
(74) Representative: Patent Boutique LLP
(86) International application number: PCT/US2013/000102
(87) International publication number: WO 2013/151595

(56) References cited:
- EP-A1- 1 093 818
- WO-A1-98/18453
- WO-A1-98/18453
- WO-A2-2004/073652
- WO-A2-2011/018504
- US-A1- 2008 152 648
- US-A1- 2008 152 648
- HAINING W N ET AL: "pH-triggered microparticles for peptide vaccination", THE JOURNAL OF IMMUNOLOGY, THE AMERICAN ASSOCIATION OF IMMUNOLOGISTS, US, vol. 173, no. 4, 15 August 2004 (2004-08-15), pages 2578-2585, XP002316975, ISSN: 0022-1767
- WON-JUNG LEE ET AL: "Efficacy of thiolated eudragit microspheres as an oral vaccine delivery system to induce mucosal immunity against enterotoxigenicin mice", EUROPEAN JOURNAL OF PHARMACEUTICS AND BIOPHARMACEUTICS, ELSEVIER SCIENCE PUBLISHERS B.V., AMSTERDAM, NL, vol. 81, no. 1, 18 January 2012 (2012-01-18), pages 43-48, XP028484391, ISSN: 0939-6411, DOI: 10.1016/J.EJPB.2012.01.010 [retrieved on 2012-01-28]
- MOREFIELD G L ET AL: "Role of aluminum-containing adjuvants in antigen internalization by dendritic cells in vitro", VACCINE, ELSEVIER LTD, GB, vol. 23, no. 13, 18 February 2005 (2005-02-18), pages 1588-1595, XP027652282, ISSN: 0264-410X [retrieved on 2005-02-18]
- LIN J H ET AL: "Protective effects of oral microencapsulated Mycoplasma hyopneumoniae vaccine prepared by co-spray drying method.", THE JOURNAL OF VETERINARY MEDICAL SCIENCE / THE JAPANESE SOCIETY OF VETERINARY SCIENCE JAN 2003, vol. 65, no. 1, January 2003 (2003-01), pages 69-74, XP002754214, ISSN: 0916-7250
- GEMMA AÑO ET AL: "A new oral vaccine candidate based on the microencapsulation by spray-drying of inactivated", VACCINE, ELSEVIER LTD, GB, vol. 29, no. 34, 27 May 2011 (2011-05-27), pages 5758-5764, XP028243926, ISSN: 0264-410X, DOI: 10.1016/J.VACCINE.2011.05.098 [retrieved on 2011-06-07]
- DIESNER, S. C. ET AL.: 'Use of lectin-functionalized particles for oral immunotherapy' THERAPEUTIC DELIVERY vol. 3, no. 2, February 2012, pages 277 - 290, XP055116139

## Description

### Cross Reference to Related Applications

This application claims priority to the filing date of US Provisional Application No. 61/686,372 filed April 4, 2012.

### Background of the Invention

Diseases such as strep throat, scarlet fever, necrotizing fasciitis, streptococcal toxic shock syndrome, and impetigo result from infection by *Streptococcus pyogenes.* These diseases are collectively referred to as Group A Streptococcal Diseases (GAS). GAS and other infections caused by *S. pyogenes* are significant source of human morbidity and mortality. Worldwide an estimated 500,000 people die annually from GAS disease. In addition to being a potentially deadly pathogen *S.pyogenes* causes an additional 600 million cases of pharyngitis each year. In the United States alone there are an estimated 10 million cases of non-invasive GAS disease and a further 11,000 cases of invasive disease with approximately 10% of these cases resulting in death. The annual cost to the US health system is estimated at $220 - 540 million per year for pediatric pharyngitis management. An effective vaccine combating *S.pyogenes* could save thousands of lives from GAS disease and allow for millions of dollars of saved medical costs. Various attempts have been made at developing efficacious vaccines against GAS and the *S. pyogenes* pathogen, but none have proven successful.

It is often necessary in vaccine development to immunologically enhance (i.e., modulate) a recipient's response to the antigens being administered that are intended to invoke a protective immune response. One of the primary technologies used in vaccine development for this purpose is to provide a chemical or biological adjuvant in addition to the antigen(s) of interest. The most well accepted compositions in this regard are the various metallic (e.g., aluminum) adjuvant compounds. Typically, the antigens of interest in the vaccine compositions are adsorb onto, or otherwise associated with, the aluminum containing adjuvant compounds.

Aluminum adjuvants have a long history of safe use in human vaccines. Their adjuvant activity is thought to arise from making the antigen particulate, causing irritation at the site of injection, and activation of the NALP3 inflammasome following internalization by antigen presenting cells (APCs). However, aluminum adjuvants alone are not always appropriate for a broad array of antigen targets because they typically stimulate a skewed Tₕ2 type immune response. It has been documented for various antigens that formulation with aluminum-containing adjuvants stimulates production of IgG1 antibodies, typical for a Tₕ2 response, while little or no IgG2a antibodies, typical of a Tₕ1 response, are produced.

US 2008/152,648 describes a composition comprising an adjuvant and a c-type lectin receptor ligand. Some compositions described are suitable for oral administration.

Therefore, there is a need to provide adjuvant compositions that have improved stability, increased potency and which are capable of providing an enhanced and mixed Tₕ1/Tₕ2 response. Moreover there is a need for orally administered vaccine /adjuvant combinations that will not be degraded in the gastrointestinal tract and target the gut associated lymphoid tissue. Orally administered vaccines also have the advantages of not needing medically trained personnel for administration and not the stringent requirements of aseptic manufacturing that injectable vaccine presentations have.

### Summary of the Invention

The present invention relates to novel adjuvant compositions and production methods for the same that enhance the immune response in a recipient (e.g., a mammal) to a broad spectrum of antigen targets. In certain of these compositions, aluminum adjuvants are associated (e.g., chemically linked) with ligands to C-type lectin (CTL) receptors. While the present invention is not intended to be limited to any particular method of action, it is contemplated that present compositions and methods provide useful improvements in the field of vaccine and therapeutics development by taking advantage of the efficiency of internalization of antigen presenting cells the proven safety of aluminum containing adjuvant compounds, combined with the ability of CTL receptor ligands to produce directed differential immune responses.

In the preferred embodiments the present invention provides adjuvant compositions that can be administered orally that have improved stability, and/or increased potency and/or provide an enhanced Tₕ1 response. The present invention also provides methods of making those compositions and methods of administration of the improved adjuvant compositions.

The present invention provides immunological compositions comprising one or more CTL receptor ligands in combination with one of more antigens and with one or more aluminum adjuvants. The CTL receptor ligand(s) in certain preferred embodiments are linked to the aluminum adjuvant(s). The CTL receptor ligand(s) can be linked by a coordinate, covalent, hydrophilic, or hydrophobic bond(s) to the aluminum adjuvant(s). Additionally, the CTL receptor ligand(s) can be linked, at least in part, through a fluoride, phosphate, sulfate, or carbonate group to the aluminum adjuvant(s).

In other preferred embodiments, the CTL receptor ligand(s) comprise monosacharides, disaccharides, and/or polysaccharides. In one aspect of the invention, these saccharides comprise a terminal end phosphate group or phosphodiester backbone.

The immunological compositions further comprise one or more metallic adjuvants comprising an aluminum adjuvant, e.g. comprising aluminum oxy hydroxide, aluminum hydroxyphosphate, aluminum hydroxyphosphate sulfate, aluminum phosphate or combinations thereof.

The immunological compositions and adjuvant systems can be administered to an animal in combination with one or more suitable vaccines against a vaccine preventable or treatable disease. The immunological compositions and adjuvant systems can be administered to an animal in combination with suitable biological products (e.g., therapeutic proteins and/or antibodies) or small molecule drug compositions. Suitable animals for administration of the adjuvants and immunological compositions of the present invention include mammals (e.g., humans) and common domesticated companion animals (e.g., dogs, cats, horses, etc.) or production/agriculturally important animals (e.g., cows, pigs, sheep, and goats).

The present invention further relates to methods of making orally administrable immunogenic compositions by adsorbing an antigen and a CTL-receptor ligand to an aluminum adjuvant, adding an acrylic resin polymer having pH dependent solubility to form a vaccine formulation and adding the vaccine formulation to a low pH solution to precipitate the polymer.

Also described are methods for formulating orally administered dry powder formulations by mixing an antigen with an acrylic resin and mannitol and sucrose in phosphate buffer to make a mixture, spraying the mixture into buffer solution at low pH to form a suspension; and drying the suspension into a powder.

Also described are methods for formulating orally administered suspensions by mixing an antigen with an acrylic resin and mannitol and sucrose in phosphate buffer to make a mixture and spraying the mixture into buffer solution at low pH to form a suspension.

### Description of the Drawings

Figure 1 is a schematic diagram which illustrates linkage of C-type lectin receptor ligands to aluminum adjuvants allows for receptor mediated endocytosis of co-localized antigen and adjuvant. In preferred embodiments, the endocytosis of co-localized antigen and adjuvant stimulates the production of Tₕ1 and Tₕ2 cytokines as well as targeting the antigen to endosomes where cross presentation of antigen on MCH I and II molecules can occur.
Figure 2 is a graph illustrating that monosaccharides exhibit low adsorption to aluminum adjuvants unless modified to contain a group capable of ligand exchange binding or polymerized.
Figure 3A is a graph illustrating that saccharides properly linked to aluminum particles remain attached to the particle even when exposed to physiological levels of phosphate in the absence of antigen.
Figure 3B is a graph illustrating that saccharides properly linked to aluminum particles remain largely attached to the particle even when exposed to physiological levels of phosphate in the presence of antigen.
Figure 4 is a graph illustrating the immunological compositions, formulated with an antigen targeting *S.pyogenes,* enhanced the production of total IgG demonstrating the potential for dose sparing.
Figure 5 is a graph illustrating that the immunological compositions, formulated with an antigen targeting *S.pyogenes*, induced production of IgG2a which is indicative of a Th2 response in rats.
Figure 6 is a graph illustrating that the immunological compositions, formulated with an antigen targeting *S.pyogenes,* induced production of IgG2b which is indicative of a Th1 response in rats.
Figure 7 is a graph of BSA at each step after coacervation of BSA and Eudragit.
Figures 8A and 8B are graphs of BSA absorption and mannan absorption after coacervation of absorbed BSA, respectively.
Figures 9A and 9B are graphs of BSA absorption and mannan absorption after coacervation of absorbed BSA, respectively after reduction of Eudragit percentages.
Figure 10 is a graph of Spe A/B absorption after coacervation of absorbed Spe A/B.

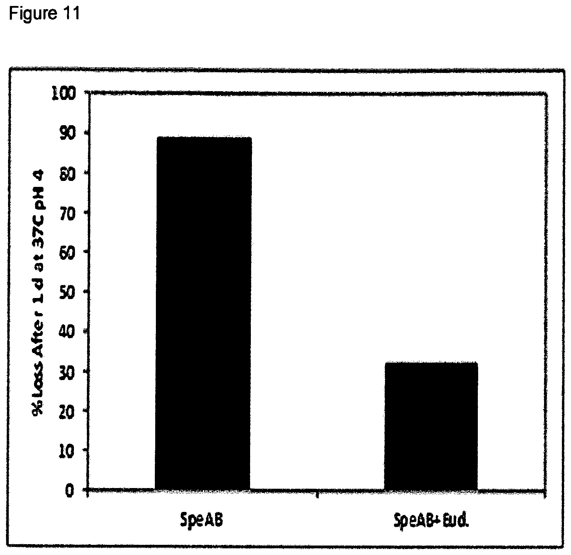

Figure 11 is a graph of Spe A/B absorption after coacervation with Eudragit L100-55.
Figure 12 is a graph of antigen specific serum total IgG at day 0, 14 and 35. Figure 13 is a graph of neutralization of wild type SpeA toxin by sera of vaccinated animals.
Figure 14. Degradation of CRM following storage at (A) 25° C and (B) 37° C.
Figure 15. Percent of CRM remaining following storage of suspension and solution at (A) 25° C and (B) 37° C.
Figure 16. Percent of CRM remaining following storage of dry powder and solution at (A) 25° C and (B) 37° C.

### Detailed Description of the Invention

The present invention provides adjuvant compositions that have improved stability and/or increased potency and/or provide an enhanced Tₕ1 response. The present invention also provides methods of making those compositions and administration of the improved adjuvant compositions.

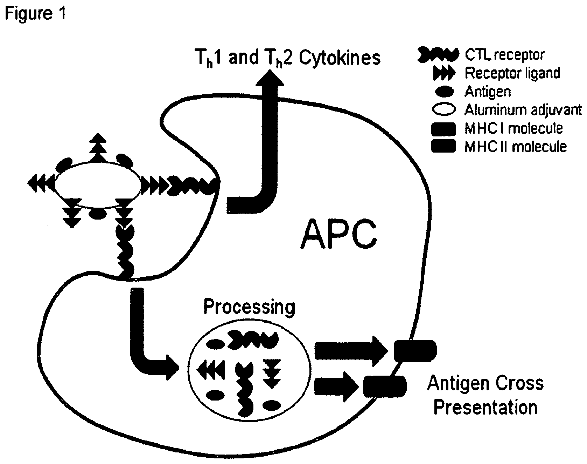

C-type lectin (CTL) receptors are located on multiple cells of the immune system including macrophages, monocytes, dendritic cells, and B-cells. CTL receptors recognize various saccharides that may be produced by pathogens. Signaling through CTL receptors can induce production of cytokines resulting in the differentiation of CD4⁺ T-cells into Tₕ1, Tₕ2, or Tₕ17 cells. Targeting of CTL receptors can also induce cross presentation of exogenous antigen on MHC I molecules and activation of CD8⁺ T-cells resulting in a cellular immune response. It has been observed that CTL receptor agonists in a soluble presentation can dampen the immune response, while CTL receptor agonists in a particulate presentation are more suitable for enhancement of immune responses. It is contemplated that by linking CTL receptor ligands to aluminum adjuvants, the mechanism of action of both compounds can be utilized to obtain a robust immune response. The aluminum-containing adjuvant acts as a delivery particle targeting APCs and ensuring the antigen and CTL receptor agonist are co-localized in the phagosome. The aluminum adjuvant and CTL receptor agonist induces Th1 and Th2 cytokines as well as MHC I and II cross presentation of the co-localized antigen resulting in a robust immune response (Figure 1)

In many instances obtaining a mixed Tₕ1/Tₕ2 immunological response provides more robust protection from disease compared to the Tₕ2 skewed response of traditional vaccine adjuvants such as aluminum oxyhydroxide. Advantages of the present invention include, but are not limited to, enhanced immunogenicity over traditional aluminum adjuvants while maintaining safety, the ability to stockpile, the ease of manufacture, and the low cost of goods for the adjuvant system. Activation of antigen presenting cells through multiple signaling pathways results in an enhanced immune response and the potential for dose sparing of antigen. Components of the adjuvant system are inherently stable allowing for stockpiling of the adjuvant under typical vaccine storage conditions. The nature, availability, and low cost of the raw materials for the adjuvant system allow for rapid manufacture of the adjuvant without specialized equipment. Therefore, in the case of emergency where the stockpile of adjuvant would need to be supplemented additional adjuvant could be supplied in a timely manner. This novel adjuvant system can play an integral role in enhancing the potency of vaccines to defend against biological attack or pandemic outbreaks of infectious agents.

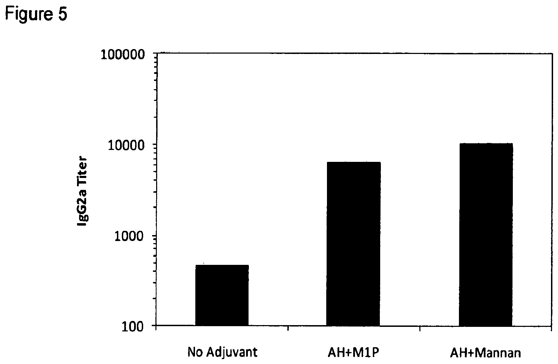

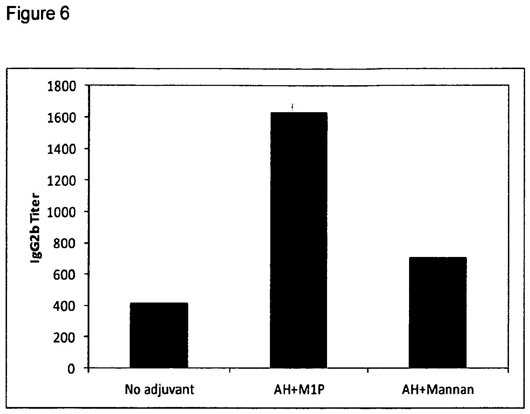

The present invention provides adjuvant compositions that provide an enhanced Tₕ1 response compared to prior art aluminum adjuvants. The combination of one or more CTL receptor agonists with one or more aluminum adjuvants where preferably the CTL receptor agonist is bound to the aluminum adjuvant increases the Th1 response relative to the aluminum adjuvant alone. The comparison of IgG2b antibodies produced by an injection of mannan bound aluminum oxyhydroxide and Spe A/B antigen, mannose-1-phosphate bound aluminum oxyhydroxide and Spe A/B antigen and aluminum oxyhydroxide without CTL receptor agonist with Spe A/B antigen when injected into rats is shown in figure 6. The increase in the IgG2b response in rats to the CTL receptor bound to aluminum oxyhydroxide corresponds to an increase in Th1 response as compared to the response for aluminum oxyhydroxide alone. Figure 5 shows an increase in the IgG2a response in rats to the CTL receptor bound to aluminum oxyhydroxide as well. This corresponds to an increase in the CTL receptor bound to aluminum oxyhydroxide Th2 response as compared to the response for aluminum oxyhydroxide alone. Thus, the adjuvant compositions of the present invention provide increased antibody responses as well as increased Th2 responses when compared to aluminum adjuvants that have not been bound to CTL receptor agonists. The aluminum compound bound CTL receptor agonist adjuvants of the present invention may provide enhanced antibody titer and/or enhanced Th2 response of greater than 5%, greater than 10%, greater than 20%, greater than 30%, greater than 50%, greater than 75% or greater than 100% over aluminum adjuvant alone. The aluminum compound bound CTL receptor agonist adjuvants of the present invention may provide enhanced antibody titer and/or enhanced Th2 response of between about 5% to about 100% or about 5% to about 90% or about 5% to about 80% or about 5% to about 70% or about 5% to about 60% or about 5% to about 50% or about 5% to about 40% or about 5% to about 30% or about 5% to about 20% or about 5% to about 10% or about 10% to about 100% or about 10% to about 80% or about 10% to about 70% or about 10% to about 60% or about 10% to about 50% or about 10% to about 40% or about 10% to about 30% or about 10% to about 20% or about 25% to about 100% or about 25% to about 75% or about 25% to about 50%.

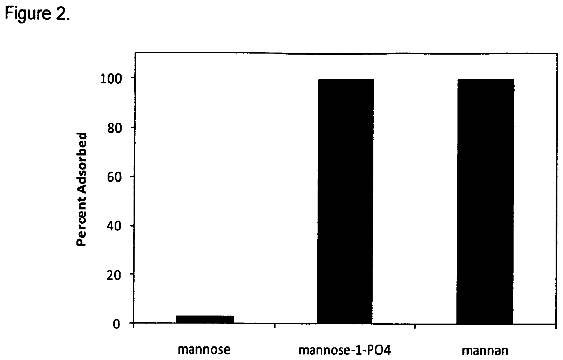

Typically small molecule CTL receptor agonists, such as monosaccharides, do not adsorb to the surface of aluminum-containing adjuvants. However, monosaccharides can be modified by a chemical group including but not limited to fluoride, phosphate, sulfate, or carbonate group which permits a ligand exchange linkage of the molecule to the aluminum-containing adjuvant. Method for modifying saccharides by addition of fluoride, phosphate, sulfate, or carbonate groups is well known in the art (Cantos, et al. Biochem. J. (1993) 288: 155-160; Carbohydrate Chemistry, Royal Society of Chemistry, Ed. RD Guthrie (1968)). This is illustrated in figure 2 utilizing mannose as the CTL receptor agonist and aluminum oxyhydroxide. The unmodified mannose has very little linkage to the aluminum. However, addition of a phosphate group at the 1 position of mannose (M1P) results in approximately 100% linkage of the CTL receptor agonist to the aluminum adjuvant.

Another method to the increased avidity of the saccharide for the aluminum adjuvant particle is polymerization. For instance the avidity of the saccharide for the aluminum adjuvant particle may be increased by increasing the size of the saccharide by polymerization to produce a polysaccharide. The increase avidity for the aluminum adjuvant is due to the larger number of interactions of a polysaccharide as compared to a monosaccharide to allow for the stable linkage of the polysaccharide agonist to the aluminum adjuvant particle. In one embodiment, the physical characterization of the adjuvant system focuses on the linkage of the CTL receptor ligand to the aluminum adjuvant as well as the stability of that linkage. As seen in Figure 2, polymerization of mannose to the polysaccharide mannan results in approximately 100% linkage of the CTL receptor agonist to the aluminum adjuvant.

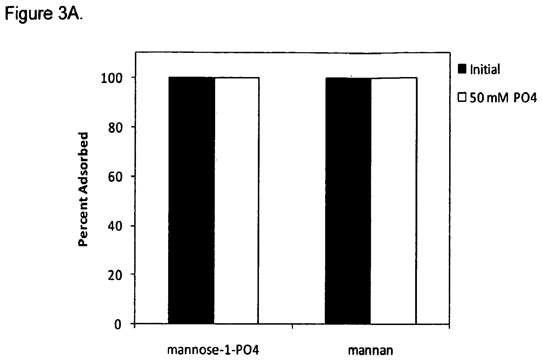

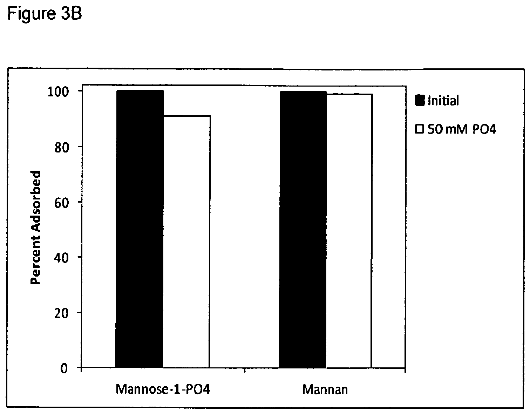

It is important to maintain the stability of the CTL receptor agonist linkage to the aluminum particle upon exposure to body fluid following administration. Components of interstitial fluid such as phosphate, citrate, and protein have the potential to remove the linkage of the CTL receptor agonist with aluminum-containing adjuvants. The resulting soluble CTL receptor agonist CTL may adversely impact the immune response. Figure 3A illustrates that M1P as well as mannan maintain approximately 100% linkage with aluminum oxyhydroxide when exposed to 50 mM phosphate pH 7.4 for 30 minutes in the absence of antigen. Figure 3B illustrates that M1P as well as mannan maintain a little less than 100% linkage with aluminum oxyhydroxide when exposed to 50 mM phosphate pH 7.4 for 30 minutes in the presence of antigen This demonstrates that linkage of the CTL receptor agonist to the aluminum particle through coordinate, covalent, hydrophilic, or hydrophobic bond is suitable to maintain the association even after administration.

Antigens used in the compositions of the present invention include viral antigens such as influenza viral antigens (e.g. hemagglutinin (HA) protein, matrix 2 (M2) protein, neuraminidase), respiratory synctial virus (RSV) antigens (e.g. fusion protein, attachment glycoprotein), polio, papillomaviral (e.g. human papilloma virus (HPV), such as an E6 protein, E7 protein, L1 protein and L2 protein), Herpes Simplex, rabies virus and flavivirus viral antigens (e.g. Dengue viral antigens, West Nile viral antigens), hepatitis viral antigens including antigens from HBV and HC. Antigens used in the compositions of the present invention include bacterial antigens including those from *Streptococcus pneumonia, Haemophilus influenza, Staphylococcus aureus, Clostridium difficile* and enteric gram-negative pathogens including *Escherichia, Salmonella, Shigella, Yersinia, Klebsiella, Pseudomonas, Enterobacter, Serratia, Proteus, B.anthracis, C.tetani, B.pertussis, S.pyogenes, S.aureus, N.meningitidis* and *Haemophilus influenzea type b.* Antigens used in the compositions of the present invention include fungal antigens including those from *Candida spp., Aspergillus spp., Crytococcus neoformans, Coccidiodes spp., Histoplasma capsulatum, Pneumocystis carinii, Paracoccidiodes brasiliensis, Plasmodium falciparum, Plasmodium vivax, Plasmodium ovale, and Plasmodium malariae.*

In one embodiment of the present invention the antigens of *Streptococcus pyogenes* (group A streptococcus, GAS) which is an important species of Gram-positive extracellular bacterial pathogen are combined in a vaccine with adjuvants of the present invention. *Streptococcal pyrogenic* exotoxin A (SpeA) and other secreted superantigen toxins are potential candidates for vaccines to prevent *S. pyogenes* infection because these proteins are associated with many outbreaks of streptococcal toxic shock syndrome and are virulence factors for invasive infections. In particular are antigens from the extracellular pyrogenic exotoxins A, B, and C. Most, *S. pyogenes* M protein serotypes express an extracellular cysteine protease (streptopain) historically termed streptococcal pyrogenic exotoxin B (SpeB), though not homologous in structure or function to SpeA or any other superantigen. Combination of these two antigens in a single fusion protein allows the immune system to eliminate infection through both toxin neutralization and bacterial opsonization. In one embodiment of the present invention Spe A/B is used as an antigen (USP 7,750,132). This SpeA/B may be composed in part of a genetically attenuated superantigen toxin protein. This purified protein product may be modified by DNA methodologies so that the superantigen attributes are absent, but the superantigen is effectively recognized by the immune system and an appropriate antibody response is produced.

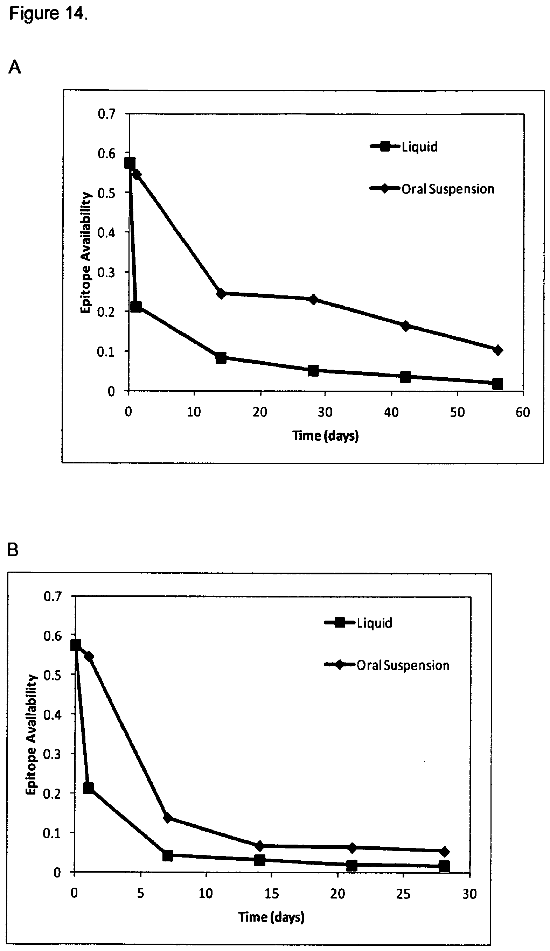

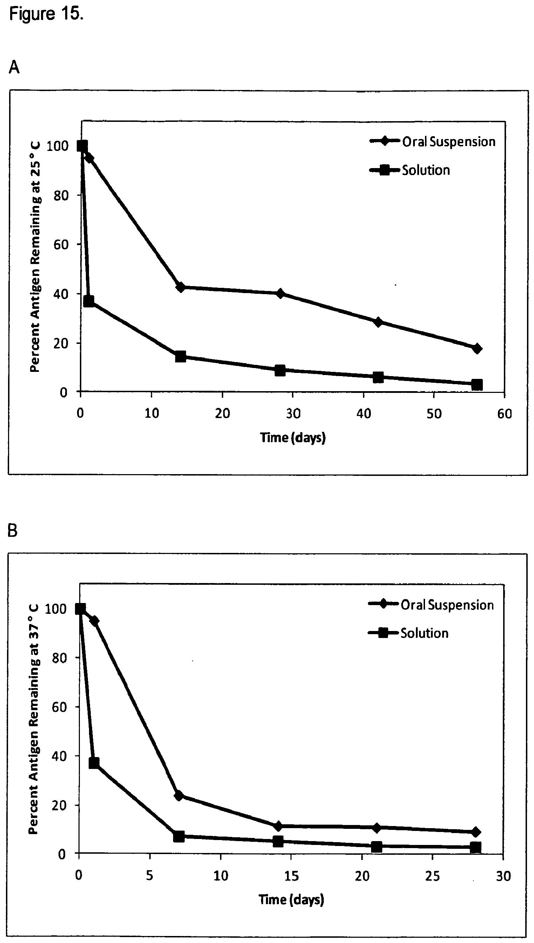

In another aspect herein described CRM₁₉₇ which is a detoxified mutant of diphtheria toxin and is used as an antigen and carrier protein in multiple vaccine formulations can be formulated as a traditional liquid and utilized as an oral vaccine formulation in either a suspension or dry powder format. The dry powder formulation may be produced through atmospheric spray freeze drying. CRM₁₉₇ is a carrier protein for conjugate vaccines against encapsulated bacteria and is currently used to vaccinate children globally against *Haemophilus influenzae,* pneumococcus, and meningococcus. The oral suspension or dry powder formulation increases the stability of CRM₁₉₇. (Fig. 14) Under the forced degradation conditions of pH 4 and increased temperature it was determined that the oral formulation was able to enhance the stability of CRM₁₉₇ versus a traditional liquid formulation. The 1^{st} order degradation rate constants were determined for both presentations at both temperatures. In both cases there is a decrease in the degradation rate in the oral vaccine presentation. This demonstrates the stabilizing effect of the oral vaccine formulation. When the oral formulation is turned into a dry powder the stability is also enhanced. There is little degradation of epitope availability after 2 months of storage at room temperature. The first order degradation rate constants were determined for each of the formulations at both temperatures. (Table 10) These results demonstrate a 68% increase in stability when CRM is formulated as an oral suspension and stored at 25° C and a 54% increase in stability when stored at 37° C. When the data is plotted as the cumulative percent of antigen lost over time it can be also be seen how the oral suspension enhances stability of CRM. (Fig. 15) It takes less than 1 day for 50% of the epitopes in the solution formulation to be lost with storage at either 25° C or 37° C. However, for the oral suspension increases the time it takes for 50% loss to 12 days at 25° C and 7 days for 37° C. The data demonstrates a significant increase in the stability of CRM₁₉₇ when formulated as an oral suspension verses a traditional liquid formulation.

The immunological compositions further comprise one or more metallic adjuvants comprising an aluminum adjuvant, e.g. comprising aluminum oxy hydroxide, aluminum hydroxyphosphate, aluminum hydroxyphosphate sulfate, aluminum phosphate, alum (potassium aluminum phosphate) or combinations thereof. In addition to aluminium, other metallic salts have been used to adsorb antigens, including salts of zinc, calcium, cerium, chromium, iron, and berilium. The hydroxide and phosphate salts of aluminium are the most common.

In other preferred embodiments, the CTL receptor ligand(s) comprise monosacharides, disaccharides, or polysaccharides. CTL receptor ligands of the present invention include saccharides which include but are not limited to Arabinose, Ribose, Ribulose, Xylose, Xylulose, Lyxose, Allose, Altrose, Fructose, Galactose, Glucose, Gulose, Idose, Mannose, Sorbose, Talose, Tagatose, Sedoheptulose, Mannoheptulose, Sucrose, Maltose, Trehalose, Lactose, Mellibiose, Amylaose, and Mannan, In one embodiment of the invention, these saccharides comprise a terminal end phosphate group or phosphodiester backbone.

Methods and scheme for administering and sufficiently dosing the immunological compositions and adjuvant systems are known within the art. The dosage and frequency (single or multiple doses) administered to a subject can vary depending upon a variety of factors, including, for example, prior exposure to an infection consequent to exposure to the antigen: health, body weight, body mass index, and diet of the subject or health-related problems. Other therapeutic regimens or agents can be used in conjunction with the methods and compositions, proteins or polypeptides of the present invention.

The immunogenic compositions for use according to the present invention may be delivered as a standard 0.5 ml injectable dose and contain from about 0.1µg to about 50µg of antigen. In a preferred embodiment of the immunogenic compositions for use according to the present invention is a standard 0.5 ml injectable dose and contains from about 3µg to about 20µg of antigen. The vaccine volume may be between 0.25 and 1.0 ml, suitably between 0.5 ml and 1.0 ml, in particular a standard 0.5ml. A vaccine dose according to the present invention may be provided in a smaller volume than conventional dosing. Low volume doses according to the present invention are suitably below 0.5ml, typically below 0.3ml and usually not less than 0.1 ml.

Currently, most vaccines are available for parental administration which makes immunization more costly and less safe, especially in developing countries. As vaccines are administered to infants, children, and adults who are generally healthy at the time of injection, there is a high level of sterility that must be ensured when manufacturing the injectable product, adding additional cost to the vaccine. Development of oral vaccine formulations has multiple advantages over parenteral injections such as reduced risk of reactogenicity, medically trained personnel are not required for administration, and reduced manufacturing cost (as the need for aseptic processing is reduced).

Oral administration is attractive from an immunological perspective as the gastrointestinal tract contains mucosal lymphoid inductive sites such as the gut-associated lymphoid tissue (GALT), which stimulate the immune system. The GI tract has over 300 m² of mucosal surface that is richly endowed with immune inductive tissue, such as Peyer's patches. In addition, mucosal vaccination has display a superior capability to induce local mucosal immune responses along with systemic vaccination since all mucosal surfaces act as the gateway sites of antigen entry. Most importantly, immunization at one mucosal sites can result in antibody secretion systemically, as well as at other selected mucosal sites [16]

The physical structures of mucosal surfaces are designed to maintain constant protection against pathogens. The microfold (M) cells are constituents of the mucosal surfaces whose function is to transport substances across the epithelial surface for subsequent uptake and processing by dendritic cells (DCs) to initiate the immune responses. These DCs prime T lymphocytes to expand clonally and differentiate into T-cell subsets (Th1, Th2, Th17, or T regulatory cells). Simultaneously, T cells are marked with mucosal homing markers that direct them to sub-mucosal regions where they perform their cell-mediated immunity functions. At this point, DCs and cognate T-cells interact with B-cells to promote their differentiation and antibody production at multiple mucosal sites. Our oral vaccine formulation takes advantage of the mucosal immune system by first providing protection against degradation of SpeAB in the stomach and then targeting the antigen to M-cells of the Peyer's patches to stimulate an immune response by the GALT. This approach provides a safe, effective, stable and economically viable vaccine for protection against GAS associated diseases in both the industrialized and developing worlds.

The immunogenic compositions for use according to the present invention may be delivered as an oral dose. Oral vaccination with the adjuvants of the present invention takes advantage of the immune tissue in the gut named peyer patches which express receptors to CTL and are most efficient at internalizing particles of the size of aluminum adjuvants. The combination of aluminum adjuvant bound to CTL receptor agonist provides surprising benefits. The CTL receptor agonist targets the vaccine particle to the M-cells of the peyer patches. The aluminum particle makes the antigen of suitable size for efficient internalization. The oral adjuvants of the present invention maintain attachment to the antigen and the adjuvant and protects the antigen from degradation in the stomach and intestines. Protection of the vaccine from degradation in the stomach may be provided by coacervating the vaccine particles with a polymers that protect the vaccine. Polymers that can be coacervated at various pHs can be obtained so suitable polymers can be paired with the appropriate protein antigens. In a one embodiment, Eudragit can be used for coacervation. Eudragit precipitates below pH 5.5 and in the Examples oral formulations containing Eudragit have lower pH than the IM formulations. The precipitated Eudragit coats the vaccine particle and protects it from degradation. Coacervation of the adjuvant with the antigen directly also can make the antigen thermostable. This could reduce reliance on cold storage for therapuetic proteins, antigen bulks, as well as final vaccine formulations.

Suitable polymers for coacervation include but are not limited to methacrylic polymers, acrylic acid and methacrylic acid copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), glycidyl methacrylate copolymers, and combinations thereof. An acrylic polymer useful for preparation of a sequestering subunit of the invention includes acrylic resins comprising copolymers synthesized from acrylic and methacrylic acid esters (e.g., the copolymer of acrylic acid lower alkyl ester and methacrylic acid lower alkyl ester) containing about 0.02 to about 0.03 mole of a tri (lower alkyl) ammonium group per mole of the acrylic and methacrylic monomer used. An example of a suitable acrylic resin is ammonio methacrylate copolymer NF21, a polymer manufactured by Rohm Pharma GmbH, Darmstadt, Germany, and sold under the Eudragit® trademark. Eudragit® is a water-insoluble copolymer of ethyl acrylate (EA), methyl methacrylate (MM) and trimethylammoniumethyl methacrylate chloride (TAM) in which the molar ratio of TAM to the remaining components (EA and MM) is 1:40. Acrylic resins, such as Eudragit®, can be used in the form of an aqueous dispersion or as a solution in suitable solvents. A preferred Eudragit in the formulations of the present invention is Eudragit L100-55. Other acrylic polymers include copolymers of acrylic and methacrylic acid esters with a low content in quaternary ammonium groups such as Eudragit® RL PO (Type A) and Eudragit® RS PO (Type B; as used herein, "Eudragit® RS") L30D55, L100, (L12,5), S100, (S12,5), and FS30D (as described the monographs Ammonio Methacrylate Copolymer Type A Ph. Eur., Ammonio Methacrylate Copolymer Type B Ph. Eur., Ammonio Methacrylate Copolymer, Type A and B USP/NF, and Aminoalkylmethacrylate Copolymer RS JPE). The selection of an appropriate Eudragit will depend on where in the gastrointenstinal track the material is to be released from the coated particle.

In one aspect, the present invention provides methods of making orally administerable immunogenic composition by adsorbing an antigen and a CTL receptor agonist to an aluminum adjuvant, adding a polymer having pH dependent solubility to form a vaccine formulation and adding the vaccine formulation to a low pH solution to precipitate the polymer. Also described are methods for formulating orally administered dry powder formulations by mixing an antigen with an acrylic resin and mannitol and sucrose in phosphate buffer to make a mixture, spraying the mixture into a solution at low pH to form a suspension; and drying the suspension into a powder. The present invention also relates to methods for formulating orally administered suspensions by mixing an antigen with an acrylic resin and mannitol and sucrose in phosphate buffer to make a mixture and spraying the mixture into a solution at low pH to form a suspension. The low pH solution any solution that can lower the pH below 5.5 and keep it there could be used. In certain embodiments acetate is preferably as it does not adversely impact the gastrointestinal track when administered and does not interact with the aluminum as other buffers like phosphate or citrate might. The pH of the solution may be 7.0 or lower or about 6.0 or lower or about 5.5 or lower, or about 5.0 or lower or about 4.5 or lower or about 4.0 or lower or about 3.5 or lower or 3.0 or lower. The pH of the solution may be between about 7.0 to about 3.0 or about 6.5 to about 3.0 or about 6.0 to about 3.0 or about 5.5 to about 3.0 or about 5.0 to about 3.0 or about 4.5 to about 3.0 or about 4.0 to about 3.0 or about 3.5 to about 3.0.

In one embodiment of the present invention an oral vaccine formulation for SpeA/B was designed using a delivery particle comprising aluminum oxyhydroxide (AIOOH). The SpeA/B was bound to the AIOOH during the formulation process, and then a targeting molecule is bound to the AIOOH particle as well. C-type lectin receptors, such as the mannose receptor, are likely present on M-cells since these are able to detect, interact and transport bacteria. C-type lectin receptor agonists, either mannose-1-phosphate (M1P) and/or polymerized mannose (Mannan), which target M-cells to promote intestinal uptake and take advantage of various binding modes with AIOOH may be utilized in the formulation. Finally, an enteric polymer is added to provide protection against degradation in the stomach. Eudragits® are pH-sensitive polymers based on poly(methacrylate); have been accepted as pharmaceutical excipients for oral use; are generally regarded as biodegradable non-toxic materials; and can protect the active pharmaceutical ingredients from degradation by enzymes and gastric juices. Eudragit® L100-55 precipitates in solutions at pH below 5.5. Therefore the vaccine is protected from the low pH of the stomach, but is released in the duodenum allowing for interaction with M-cells in the intestines. In addition to adding excipients that protect the antigen(s) from gastric juices the compositions of the present invention can also include an encapsulation process to protect the antigen(s) such as SpeA/B. The immune response will be enhanced if the integrity of the antigen is maintained in the GI, prior to releasing to the Payer's patches. Coacervated of the vaccine particles will guarantee a more sophisticated armor around the immunogenic protein, in addition to utilization of new formulation technologies for the oral administration of vaccines which will also address effectiveness in storage and transportation, as well as safety in needle-free vaccines. One of those formulation technologies includes spray freeze drying processes to effectively coat the vaccine compositions. Spray freeze drying (SFD) is known to enable the production of powder particles with well-defined physical properties including low density, high surface area, well defined particle size distribution and potentially very rapid dissolution. The present invention relates to vaccine particles coated with an atmospheric spray freeze drying process (ASFD) which can confer desirable particle physical properties, though with reduced risk of thermal and pressure differential damage to sensitive, antigenic structure. ASFD also has the advantage of lending itself to large scale continuous processing.

During ASFD a carefully formulated liquid solution is atomized to a specifically sized spherical droplet and immediately frozen, locking in the size and shape of each individual particle. The particles are then dried by passing a cryogenic gas (nitrogen) through the particle bed. The flow and temperature profiles can be customized to give the particles the desired morphology for their eventual application. Because of the use of convective heat transfer, the process is usually much quicker than lyophilization and the elimination of the need for high vacuum lowers cost and facilitates transition to a manufacturing scale. Thus far there are only a few examples in the literature where lyophilization, spray drying, and spray freeze drying protocols have been employed to prepare controlled-release solid-dosage for oral delivery of therapeutics, and in a fewer cases, of vaccines. Spray drying has been successful in preparing well defined particles, but this technology requires the application of heat which may affect the potency of immunogenic proteins by causing denaturation. Lyophilization and spray freeze drying methodologies employ cryoprotectants to stabilize the 3D structure of proteins during the drying process. However, lyophilization forms particles with irregular shape and have non-homogeneous drug-to-polymer distributions, which can cause undesirable release profiles. Spray freeze drying (SFD) methods are useful in producing protein-based dry particles, but ASFD method is superior as it does not expose sensitive proteins to the stresses of major differential pressures in the processing. There are a variety of possible cryoprotectants that can be used including but not limited to mannitol, lactose, sorbitol, and sucrose, and combinations thereof. ASFD utilizes atomizing nozzles which are used to produce ideal particle physical properties, such as uniform coacervation of the formulation.

The compositions of the present invention can be administered alone or as admixtures with conventional excipients, for example, pharmaceutically, or physiologically, acceptable organic, or inorganic carrier substances suitable for enteral or parenteral application which do not deleteriously react with the composition. Suitable pharmaceutically acceptable carriers include water, salt solutions (such as Ringer's solution), alcohols, oils, gelatins and carbohydrates such as lactose, amylose or starch, fatty acid esters, hydroxymethylcellulose, and polyvinyl pyrolidine. Such preparations can be sterilized and, if desired, mixed with auxiliary agents such as lubricants, preservatives, stabilizers, wetting agents, emulsifiers, salts for influencing osmotic pressure, buffers, coloring and/or aromatic substances and the like which do not deleteriously react with the compositions administered to the human. Preferred diluents for diluting the vaccines of the present invention include but are not limited to 150mM NaCl with histidine and trehalose.

The determination of the appropriate amount of adjuvant combined with the CTL receptor ligand will depend on a variety of factors including the type of adjuvant, CTL receptor ligand as well as the antigen in the formulation. In fact, the amount of absorptive capacity of the CTL receptor ligand used will define the upper limit of the amount of adjuvant that can be absorbed. In the compositions and methods of the present invention the amount of saccharide to adjuvant may be in the range of about 1.5 mg of saccharide/ 1 mg adjuvant to about 0.05 mg of saccharide/ 1 mg adjuvant. In other embodiments of the compositions and methods of the present invention the amount of saccharide to adjuvant may be in the range of about 1.25 mg of saccharide/ 1 mg adjuvant to about 0.05 mg of saccharide/ 1 mg adjuvant about 1.0 mg of saccharide/ 1 mg adjuvant to about 0.05 mg of saccharide/ 1 mg adjuvant or about 1.0 mg of saccharide/ 1 mg adjuvant to about 0.05 mg of saccharide/ 1 mg adjuvant, or about 0.5 mg of saccharide/ 1 mg adjuvant to about 0.05 mg of saccharide/ 1 mg adjuvant or about 1.5 mg of saccharide/ 1 mg adjuvant to about 0.10 mg of saccharide/ 1 mg adjuvant or about 1.5 mg of saccharide/ 1 mg adjuvant to about 0.25 mg of saccharide/ 1 mg adjuvant or about 1.5 mg of saccharide/ 1 mg adjuvant to about 0.50 mg of saccharide/ 1 mg adjuvant or about 1.5 mg of saccharide/ 1 mg adjuvant to about 0.75 mg of saccharide/ 1 mg adjuvant.

The determination of the appropriate amount of antigen combined with the adjuvant/CTL receptor ligand will depend on a variety of factors including the type of adjuvant, CTL receptor ligand as well as the antigen in the formulation. In the compositions and methods of the present invention the amount of saccharide/adjuvant to antigen may be in the range of about 1.5 mg of saccharide/adjuvant per 1 mg of antigen to about 0.05 mg of saccharide/adjuvant per 1 mg of antigen. In other embodiments of the compositions and methods of the present invention the amount of saccharide to adjuvant may be in the range of about 1.25 mg of saccharide/adjuvant per 1 mg antigen to about 0.05 mg of saccharide/adjuvant per 1 mg antigen or about 1.0 mg of saccharide/adjuvant per 1 mg antigen to about 0.05 mg of saccharide/adjuvant per 1 mg antigen or about 1.0 mg of saccharide/adjuvant per 1 mg antigen to about 0.05 mg of saccharide/adjuvant per 1 mg antigen or about 0.5 mg of saccharide/adjuvant per 1 mg antigen to about 0.05 mg of saccharide/adjuvant per 1 mg antigen or about 1.5 mg of saccharide/adjuvant per 1 mg antigen to about 0.10 mg of saccharide/adjuvant per 1 mg antigen or about 1.5 mg of saccharide/adjuvant per 1 mg antigen to about 0.25 mg of saccharide/adjuvant per 1 mg antigen or about 1.5 mg of saccharide/adjuvant per 1 mg antigen to about 0.50 mg of saccharide/adjuvant per 1 mg antigen or about 1.5 mg of saccharide/adjuvant per 1 mg antigen to about 0.75 mg of saccharide/adjuvant per 1 mg antigen.

The formulations and methods of the present invention provide vaccine formulations which are more stable that formulations made by conventional means. For example the oral suspension formulations of the present invention are at least 10% more or are at least 20% more or are at least 30% more or are at least 40% more or are at least 50% more or are at least 60% more or are at least 70% more or are at least 80% more or are at least 90% more or are at least 100% more stable than solutions with similar components. The oral suspension formulations of the present invention are about 5% to 500% more stable or about 5% to about 100% or about 5% to 90% more stable or about 5% to about 80% or about 5% to 70% more stable or about 5% to about 60% or about 5% to 50% more stable or about 5% to about 40% or about 5% to 30% more stable or about 5% to about 20% or about 5% to 10% more stable or about or about 10% to 500% more stable or about 10% to about 100% or about 10% to 90% more stable or about 10% to about 80% or about 10% to 70% more stable or about 10% to about 60% or about 10% to 50% more stable or about 10% to about 40% or about 10% to 30% more stable or about 10% to about 20% or about 25% to 500% more stable or about 25% to about 100% or about 25% to 90% more stable or about 25% to about 80% or about 25% to 70% more stable or about 25% to about 60% or about 25% to 50% more stable or about 25% to about 40% or about 50% to 500% more stable or about 50% to about 100% or about 50% to 90% more stable or about 50% to about 80% or about 50% to 70% more stable or about 50% to about 60% or about 75% to 500% more stable or about 75% to about 250% or about 75% to 100% more stable than solutions with similar components.

For example the dry powder formulations of the present invention are at least 10% more or are at least 20% more or are at least 30% more or are at least 40% more or are at least 50% more or are at least 60% more or are at least 70% more or are at least 80% more or are at least 90% more or are at least 100% more stable than solutions with similar components. The oral suspension formulations of the present invention are about 5% to 500% more stable or about 5% to about 100% or about 5% to 90% more stable or about 5% to about 80% or about 5% to 70% more stable or about 5% to about 60% or about 5% to 50% more stable or about 5% to about 40% or about 5% to 30% more stable or about 5% to about 20% or about 5% to 10% more stable or about or about 10% to 500% more stable or about 10% to about 100% or about 10% to 90% more stable or about 10% to about 80% or about 10% to 70% more stable or about 10% to about 60% or about 10% to 50% more stable or about 10% to about 40% or about 10% to 30% more stable or about 10% to about 20% or about 25% to 500% more stable or about 25% to about 100% or about 25% to 90% more stable or about 25% to about 80% or about 25% to 70% more stable or about 25% to about 60% or about 25% to 50% more stable or about 25% to about 40% or about 50% to 500% more stable or about 50% to about 100% or about 50% to 90% more stable or about 50% to about 80% or about 50% to 70% more stable or about 50% to about 60% or about 75% to 500% more stable or about 75% to about 250% or about 75% to 100% more stable than solutions with similar components. The stability of the formulations may be measured at a variety of temperatures including 25°C and 37°C. The stability of the formulations may be measured in various ways including but not limited to epitope availability,

### Example 1

### Vaccine formulations

Vaccine compositions were formulated as described in Table 1.

**Table 1 - Vaccine formulations**

| Lot # | Formulation |
|---|---|
| 11VF001 | 100µg SpeA/B, 20mM Tris, 130 mM NaCl |
| 11VF002 | 100µg SpeA/B, 20mM Tris, 130 mM NaCl, 1.7 mg/ml AH |
| 11VF003 | 100µg SpeA/B, 20mM Tris, 130 mM NaCl, 1.7 mg/ml AH, 300µg/ml mannose-1-P |
| 11VF004 | 100µg SpeA/B, 10mM Tris, 7mM NaCl, 1.7 mg/ml AH, 300µg/ml mannose-1-P, 0.1% Eudragit, 75mM sodium acetate |
| 11VF005 | 100µg SpeA/B, 20mM Tris, 130 mM NaCl, 1.7 mg/ml AH, 300µg/ml mannan |
| 11VF006 | 100µg SpeA/B, 10mM Tris, 7mM NaCl, 1.7 mg/ml AH, 300µg/ml mannan, 0.1% Eudragit, 75mM sodium acetate |
| 11VF007 | 20mM Tris, 130 mM NaCl, 1.7 mg/ml AH, 300µg/ml mannose-1-P |
| 11VF008 | 20mM Tris, 130 mM NaCl, 1.7 mg/ml AH, 300µg/ml mannan |

**Table 2 - components of Formulation 11VF003**

| Components | Concentration | Quantity |
|---|---|---|
| Spe A/B | 1.9 mg/ml | 0.297ml |
| alhydrogel | 10 mg/ml | 0.383ml |
| Mannose-1-phosphate | 4 mg/ml | 0.169ml |
| Tris buffer | 50mM | 0.714ml |
| NaCl | 1M | 0.293ml |
| Water | --- | 0.394ml |
| Total | | 2.25ml |

Water, tris and alhydrogel were placed into a 15ml tube and mixed with a vortex mixer. Spe A/B was added into the 15ml tube and mixed by vortexing. The mixture was incubated at room temperature for 30 minutes. Mannose-1-phosphate was added followed by the addition of 1M NaCl. The entire mixture was mixed by vortexing and the mixture was incubated for 30 minutes at room temperature.

**Table 3 - components of Formulation 11VF005**

| Components | Concentration | Quantity |
|---|---|---|
| Spe A/B | 1.9 mg/ml | 0.297ml |
| alhydrogel | 10 mg/ml | 0.383ml |
| Mannan | 4 mg/ml | 0.169ml |
| Tris buffer | 50mM | 0.714ml |
| NaCl | 1M | 0.293ml |
| Water | --- | 0.394ml |
| Total | | 2.25ml |

Water, tris and alhydrogel were placed into a 15ml tube and mixed with a vortex mixer. Spe A/B was added into the 15ml tube and mixed by vortexing. The mixture was incubated at room temperature for 30 minutes. Mannan was added followed by the addition of 1M NaCl. The entire mixture was mixed by vortexing and the mixture was incubated for 30 minutes at room temperature.

### Example 2

### Stability of the Vaccine Formulations

| Form. | Assay | Day | | Target | Target met |
|---|---|---|---|---|---|
| | | 0 | 21 | | |
| 11VF001 | Visual inspection | conform | conform | Clear, colorless, solution free of external particles | yes |
| | pH | 7.48 | 7.51 | pH between 7 and 8 | Yes |
| 11VF002 | Visual inspection | conform | conform | Uniform white, opaque suspension free of external particles | Yes |
| | pH | 7.19 | 7.14 | pH between 7 and 8 | Yes |
| | % SpeA/B adsorbed | 95% | 94% | >80% adsorbed | Yes |
| | % Spe A/B desorbed | 32% | 23% | % desorption steady over study | No |
| 11VF003 | Visual inspection | conform | conform | Uniform white, opaque suspension free of external particles | Yes |
| | pH | 7.27 | 7.21 | pH between 7 and 8 | Yes |
| | % SpeA/B adsorbed | 94% | 71% | >80% adsorbed | No |
| | % Spe A/B desorbed | 32% | 48% | % desorption steady over study | No |
| | %M1P adsorbed | 100% | 81% | >80% adsorbed | Yes |
| | %M1p desorbed | 9% | 19% | % desorption steady over study | No |
| 11VF004 | Visual inspection | conform | conform | Uniform white, opaque suspension free of external particles | Yes |
| | pH | 4.16 | 4.27 | pH less than 5.5 | Yes |
| | % SpeA/B adsorbed | 100% | 76% | >80% adsorbed | No |
| | % Spe A/B desorbed | 20% | 34% | % desorption steady over study | No |
| | %M1P adsorbed | 84% | 78% | >80% adsorbed | Yes |
| | %M1p desorbed | 24% | 22% | % desorption steady over study | Yes |
| 11VF005 | Visual inspection | conform | conform | Uniform white, opaque suspension free of external particles | Yes |
| | pH | 7.17 | 7.12 | pH between 7 and 8 | Yes |
| | % SpeA/B adsorbed | 96% | 93% | >80% adsorbed | Yes |
| | % Spe A/B desorbed | 30% | 23% | % desorption steady over study | Yes |
| | %mannan adsorbed | 100% | 100% | >80% adsorbed | Yes |
| | %mannan desorbed | 1% | 0% | % desorption steady over study | Yes |
| 11VF006 | Visual inspection | conform | conform | Uniform white, opaque suspension free of external particles | Yes |
| | pH | 4.16 | 4.24 | pH less than 5.5 | Yes |
| | % SpeA/B adsorbed | 96% | 96% | >80% adsorbed | Yes |
| | % Spe A/B desorbed | 22% | 12% | % desorption steady over study | No |
| | %mannan adsorbed | 100% | 100% | >80% adsorbed | Yes |
| | %mannan desorbed | 0% | 0% | % desorption steady over study | Yes |

Determination of the amount of carbohydrate in a sample was performed as described below. 50 µl of blank, standard, and sample were pipetted into the appropriate wells of a 96-well microplate. 150 µl of concentrated sulfuric acid was pipette into each well followed by pipetting of 30 µl of 5% phenol into each well. The plate was incubated at 90° C for 5 min and then cooled to room temperature. The absorbance at 490 nm was then measured. In cases where the sample had a carbohydrate concentration greater than the highest standard, dilutions of the sample were prepared such that the results fall in the linear range of the assay.

Determination of the percent antigen absorbed in the adjuvanted formulation was performed as follows. The samples were centrifuged for 5 minutes at 10,000 rcf. Working BCA reagent was prepared and the reagents were mixed in a 50:1 ratio of A to B. 25 µl of standards and samples were pipetted in triplicate in the appropriate wells of a 96 well plate and 200 µl of BCA reagent was added to each well. The plate was incubated at 37° C for 30 minutes and then cooled to room temperature. The absorbance of the wells in plate was read with a microplate reader at 570 nm. The antigen concentration in each sample using the standard curve.

Determination of the percent antigen desorbed in the adjuvanted formulation was performed as follows. The samples were centrifuged for 5 minutes at 10,000 rcf. The supernatant was removed and stored in a microcentrifuge tube. The pellet was resuspended in desorption buffer with an equivalent volume to the amount of supernatant removed and incubated for 30 minutes at room temperature. Working BCA reagent was prepared and reagents were mixed in a 3:1 ratio of A to B. The samples were centrifuged for 5 minutes at 10,000 rcf. 50 µl of standards and samples in triplicate were pipetted in the appropriate wells of a 96 well plate and 150 µl of BCA reagent was added to each well. The plate was incubated at 37° C for 30 minutes and then allowed to cool to room temperature. The absorbance of the wells in the plate was measured with the microplate reader at 570 nm and the antigen concentration in each sample was calculated using the standard curve.

### Example 3

### Adjuvant System Activity In Vivo

The potency of the adjuvant system was evaluated *in vivo* in established animal models for human pathogens.

**Table 4 - Antigen/adjuvant formulations for in vivo testing**

| Lot # | Formulation | Route of delivery | Total volume |
|---|---|---|---|
| 11VF001 | 100µg SpeA/B, 20mM Tris, 130 mM NaCl | IM | 2.25 ml |
| 11VF002 | 100µg SpeA/B, 20mM Tris, 130 mM NaCl, 1.7 mg/ml AH | IM | 2.25 ml |
| 11VF003 | 100µg SpeA/B, 20mM Tris, 130 mM NaCl, 1.7 mg/ml AH, 300µg/ml mannose-1-P | IM | 2.25 ml |
| 11VF004 | 100µg SpeA/B, 10mM Tris, 7mM NaCl, 1.7 mg/ml AH, 300µg/ml mannose-1-P, 0.1% Eudragit, 75mM sodium acetate | ORAL | 7.8 ml |
| 11VF005 | 100µg SpeA/B, 20mM Tris, 130 mM NaCl, 1.7 mg/ml AH, 300µg/ml mannan | IM | 2.25 ml |
| 11VF006 | 100µg SpeA/B, 10mM Tris, 7mM NaCl, 1.7 mg/ml AH, 300µg/ml mannan, 0.1% Eudragit, 75mM sodium acetate | ORAL | 7.8 ml |
| 11VF007 | 20mM Tris, 130 mM NaCl, 1.7 mg/ml AH, 300µg/ml mannose-1-P | IM | 4 ml |
| 11VF008 | 20mM Tris, 130 mM NaCl, 1.7 mg/ml AH, 300µg/ml mannan | IM | 4 ml |

| | | | |
|---|---|---|---|
| AH- aluminum oxyhydroxide | | | |

Formulations 11VF001-008 were tested in rats by immunizing the rats intramuscularly or orally as described in Table 4 at day 0 and day 21. Sera was collected from the rats at day -7, day 14 and day 35 and assayed for antigen specific total IgG as well IgG2a and IgG2b.

**Table 5 - Log of Total IgG Titer for individual rats at day 14**

| | Log Total IgG Titer (day 14) | | | | | |
|---|---|---|---|---|---|---|
| Individual | 11VF001 | 11VF002 | 11VF003 | 11VF004 | 11VF005 | 11VF006 |
| 1 | 4.29 | 4.67 | 4.99 | 2.18 | 5.27 | 1.70 |
| 2 | 3.66 | 4.93 | 5.17 | 1.7 | 5.67 | 1.70 |
| 3 | 3.66 | 5.25 | 5.00 | 2.36 | 5.48 | 2.31 |
| 4 | 3.64 | 4.98 | 5.04 | 1.70 | 5.24 | 3.15 |
| 5 | 3.47 | 5.31 | 5.11 | 1.70 | 5.10 | 1.70 |
| Avg | 3.74 | 5.03 | 5.06 | 1.93 | 5.35 | 2.11 |

**Table 6 - Log of Total IgG Titer for individual rats at day 35**

| | Log Total IgG Titer (day 35) | | | | | |
|---|---|---|---|---|---|---|
| Individual | 11VF001 | 11VF002 | 11VF003 | 11VF004 | 11VF005 | 11VF006 |
| 1 | 6.02 | 5.88 | 6.25 | 2.59 | 6.09 | 2.83 |
| 2 | 5.77 | 6.13 | 6.34 | 2.16 | 6.42 | 1.70 |
| 3 | 5.58 | 6.24 | 6.41 | 2.11 | 6.23 | 3.33 |
| 4 | 6.05 | 6.35 | 6.39 | 1.70 | 6.20 | 2.12 |
| 5 | 5.62 | 6.24 | 6.28 | 1.70 | 6.28 | |
| Avg | 5.81 | 6.16 | 6.33 | 2.05 | 6.24 | 2.50 |

**Table 7 - Mean Log of Total IgG Titer days 14, 35 with standard deviation**

| | | Group | | | | | |
|---|---|---|---|---|---|---|---|
| Mean Log Titer | Day | 11VF001 | 11VF002 | 11VF003 | 11VF004 | 11VF005 | 11VF006 |
| | 0 | 1.70 | 1.70 | 1.70 | 1.70 | 1.70 | 1.70 |
| | 14 | 3.74 | 5.03 | 5.06 | 1.93 | 5.35 | 2.11 |
| | 35 | 5.81 | 6.16 | 6.33 | 2.05 | 6.24 | 2.50 |
| Std dev. | 14 | 0.31 | 0.26 | 0.08 | 0.32 | 0.22 | 0.48 |
| | 35 | 0.21 | 0.18 | 0.07 | 0.37 | 0.12 | 0.60 |

To determine the antibody titer the following procedure was performed. A 2 µg/ml solution of coating antigen in coating buffer was prepared. 100 ml of 2 µg/ml antigen was placed in each well of a 96 well plate and incubated for 1 hour at 37° C. The plate was washed once with 100 µl/well of washing buffer and 100 µl of blocking buffer was pipette into each well and incubate the plate for 1 hour at 37° C. Two-fold serial dilutions of the unknown sera in washing buffer were prepared. For early time points the dilution was typically a 1:50 dilution. For later time points the dilution was at 1:10,000 or higher. The plate was washed twice with 100 µl/well of washing buffer. 100 µl of each sera dilution in duplicate was pipetted into the appropriate plate wells and incubate the plate for 1 hour at 37° C. An appropriate dilution of the detection antibody in blocking buffer was prepared. The plate was washed three times with 100 µl/well of washing buffer and 100 µl of detection antibody was pipette into each well and incubate the plate for 1 hour at 37° C. The plate was washed three times with 100 µl/well of washing buffer. An appropriate volume of TMB working reagent was prepared by combining 1 part solution A with 1 part solution B. 100 µl of TMB was pipetted into each well and incubate the plate for 15 minutes at room temperature. If the plate had not completed development the incubation time was increased in 5 minute increments until color developed. 100 µl of 3 M sulfuric acid was pipette into each well to stop the reaction and the absorbance at 450 nm was measured for each well. The titer was determined as the point of the 4 parameter best fit curve that is equivalent to twice the background absorbance.

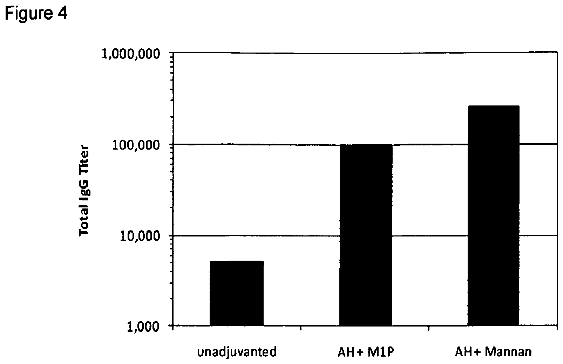

Figure 4 illustrates that these embodiments of the adjuvant system were able to increase the total serum IgG by over one log compared to unadjuvanted antigen. This demonstrates the potential for antigen dose sparing through utilization of the adjuvant system. Figure 5 illustrates that these embodiments of the adjuvant system enhanced the production of IgG2a antibodies. As the total IgG and IgG2a titers were not equivalent the immune response is mixed Th1/Th2 in nature. Figure 6 illustrates that these embodiments of the adjuvant system enhanced the production of IgG2b antibodies. As the total IgG and IgG2b titers were not equivalent the immune response is mixed Th1/Th2 in nature.

### Example 4

### Coacervation of a BSA Solution

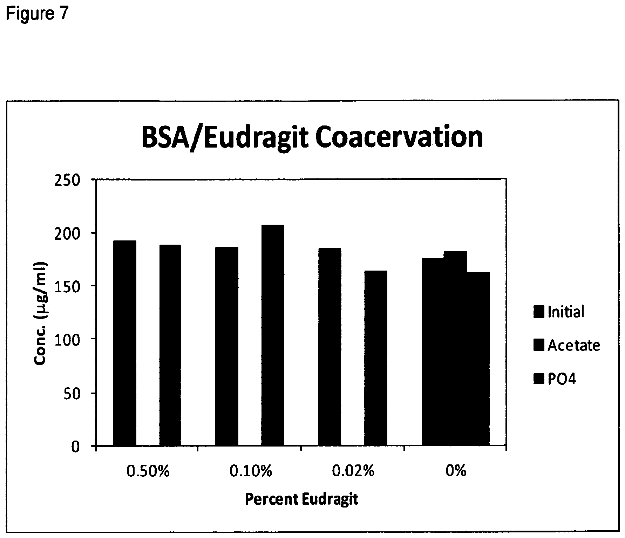

Eudragit at 0.5, 0.1 and 0.02% was added to a 200µg/ml solution of bovine serum albumin (BSA) in 800µg/ml mannan, 20 mM Tris at pH 7.4. The solution was added dropwise to 150 mM acetate buffer at pH 4 to precipitate the Eudragit. Precipitated particles were centrifuged, supernatant removed and reconstituted in 50 mM phosphate buffer to dissolve the Eudragit. The amount of BSA in solution at each step in the process was monitored to determine whether the BSA was encapsulated in the Eudragit. Figure 7 shows the results of the test.

### Example 5

### Coacervation of Adsorbed BSA1

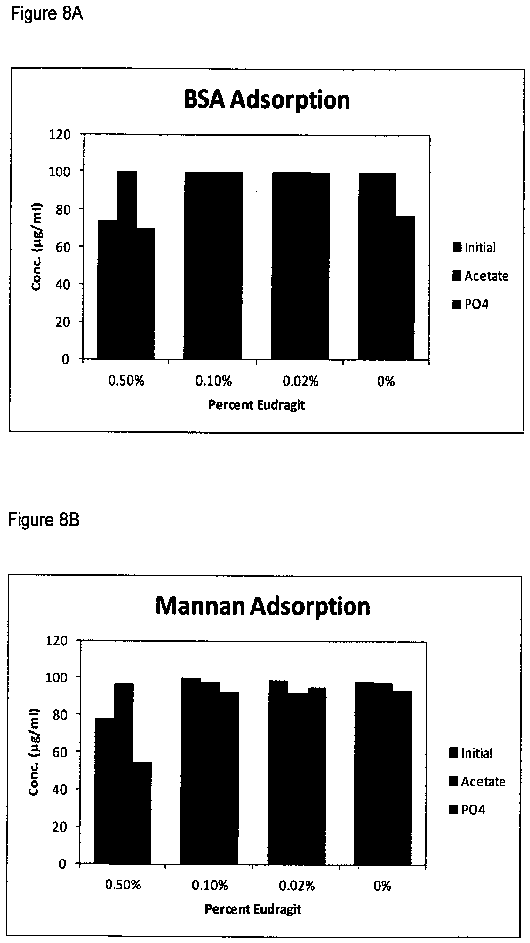

Example 4 was repeated however, the BSA was adsorbed to mannan-alhydrogel. Eudragit at 0.5, 0.1 and 0.02% was added to a 200µg/ml solution of bovine serum albumin (BSA) in 800µg/ml mannan, 3.4 mg/ml alhydrogel. Figures 8A and 8B shows the results of the test.

### Example 6

### Coacervation of Adsorbed BSA2

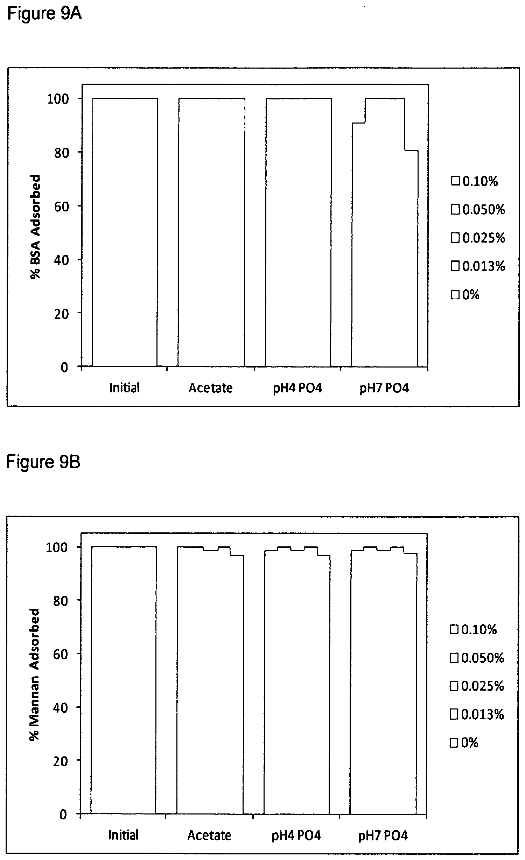

Example 5 was repeated however this time the amount of Eudragit was reduced. Eudragit at 0.1, 0.05, 0.025, 0.13% was added to a 200µg/ml solution of bovine serum albumin (BSA) in 800µg/ml mannan, 3.4 mg/ml alhydrogel. Figures 9A and 9B show the results of the test.

### Example 7

### Coacervation of Adsorbed Spe A/B

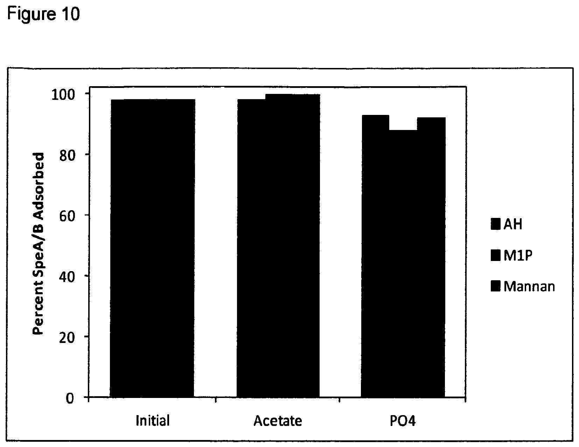

Example 6 was repeated except 100 mg/ml SpeA/B, 0.1% Eudragit, 3.4 mg/ml Alhydrogel, 20 mM Tris, and 600 mg/ml either M1P or Mannan were added together. SpeA/B was adsorbed to half of the total aluminum. The remaining aluminum was added to the acetate..This was done to provide protection from enzyme degradation in the intestine. Trypsin and Chymotrypsin should adsorb to the aluminum and be less active. Data in figure 10 demonstrated that nearly all of the SpeA/B remained adsorbed to the adjuvant.

### Example 8

### Coacervation of Spe A/B with Eudragit L100-55

200 µg/ml of protein, with or without 0.1% Eudragit® L100-55, 20 mM Tris at pH 7.5 was added dropwise to an equivalent volume of 150 mM sodium acetate pH 4. Samples were stored for 24 hr at 37° C. Samples were were dissolved in 50 mM phosphate buffer. 100 µl of serial dilutions of sample and standards in 10 mM PO₄, 150 mM NaCl were added to a 96 well plate and incubated for 1 hour at 37° C. The plate was washed with 10 mM PO₄, 150 mM NaCl, 0.05% Tween 20. 100 µl of 10 mM PO₄, 150 mM NaCl, 1% BSA was added to each well and incubated for 1 hour at 37° C. The plate was washed again. 100 µl of a 1:25,000 dilution of anti-SpeAB rat sera was added to each well and incubated for 1 hour at 37° C. The plate was washed again. 100 µl of a 1:75,000 dilution of anti-rat IgG-HRP was added to each well and incubated for 1 hour at 37° C. The plate was washed again. 100 µl of TMB was added to each well and incubated at room temperature for 15 min. The reaction was stopped with 100 µl of 3M H₂SO₄ and the absorbance was read at 450nm. The concentration of each sample was calculated from the standard curve.

### Example 9

### Antibody testing

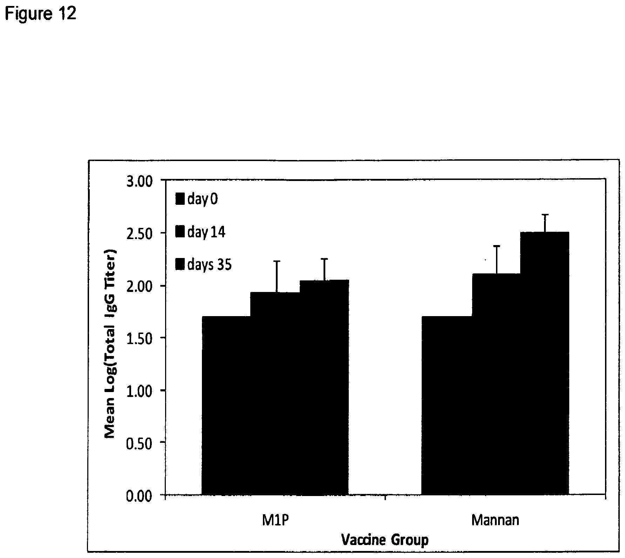

Antigen specific serum total IgG was determined for individual rats in each group at day 0, 14, and 35 of the study. 100 µl of 2µg/ml SpeAB in 10 mM PO4, 150 mM NaCl was added to a 96 well plate and incubated for 1 hour at 37° C. The plate was washed with 10 mM PO4, 150 mM NaCl, 0.05% Tween 20. 100 µl of 10 mM PO4, 150 mM NaCl, 1% BSA was added to each well and incubated for 1 hour at 37° C. The plate was washed again. Sera was serially diluted on the plate starting at 1:50 in 2 fold dilutions and incubated for 1 hour at 37° C. The plate was washed again. 100 µl of a 1:75,000 dilution of anti-rat IgG-HRP was added to each well and incubated for 1 hour at 37° C. The plate was washed again. 100 µl of TMB was added to each well and incubated at room temperature for 15 min. The reaction was stopped with 100 µl of 3M H₂SO₄ and the absorbance was read at 450nm. The concentration of each sample was calculated from the standard curve. Results are shown in Figure 12.

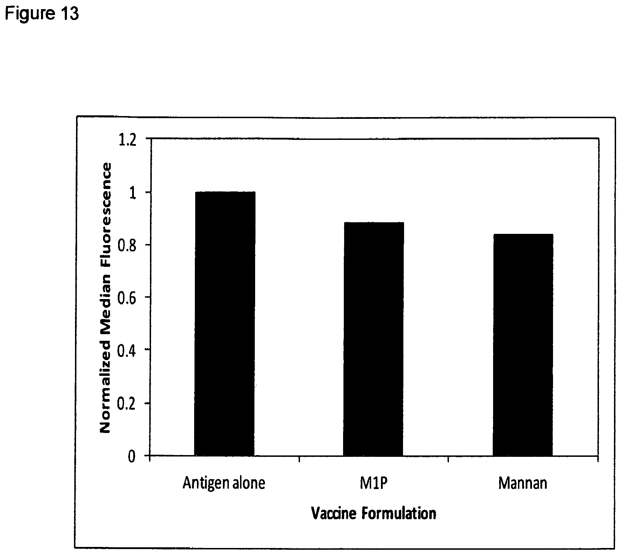

Neutralization of wild type SpeA toxin by sera of vaccinated animals. Sera was diluted 1:500 and combined with 400 ng/ml of SpeA toxin. The mixture was incubated for 1 hour at 37° C. 50 µl of the sera/toxin mixture was added to 2.5x10⁶ cells per well of human PBMCs and incubated for 24 hour at 37° C. Supernatant was collected and assayed for INF-γ production utilizing a fluorescence microarray. Median fluorescence intensity was normalized against control rat serum. Results are shown in Figure 13.

### Example 10

### AFSD Coating

Various antigens including Spe A/B, alhydrogel, mannan, and Eudragit L100-55 polymer, in conjunction with various cryoprotectants are sprayed with various atomizing nozzles into a liquid nitrogen bath to produce and preserve formed droplets. The droplets are sublimated and dried by the ASFD process. The physical properties of the dried solid particles are characterized by particle size distribution (laser diffraction), protein content and uniformity (UV spectroscopy, IR, HPLC), and morphology (helium ion microscopy and SEM).

### Example 11

### AFSD Coating 2

Enteric (Eudragit L100-55) polymer is precipitated in a buffer (pH 4.0) around various immunogens (including Spe A/B and various cyroprotectants are added to the precipitated immunogen colloidal solution and the solution is atomized, dried and characterized as in Example 10.

### Example 12

### AFSD coating 3

This example combines the encapsulation of the immunogen in two pH sensitive enteric polymers for testing of a prolonged GI releasing mechanism. Antigen, (including Spe A/B) Alhydrogel®, mannan, and an enteric polymer that precipitates at pH< 7.0, Eudragit L100, is atomized and sprayed into a liquid buffer at pH 6.0. Eudragit L100-55 is added to this colloidal suspension and the resultant suspension is atomized and sprayed into a liquid buffer at pH 4.0. At this point, the colloidal suspension is combined with a cryoprotectant and sublimed and dried by the ASFD process and the resultant particles characterized as in Example 10.

### Example 13

### GI Degradation Assessment

Formulations produced in Examples 10-12 are exposed to simulated gastric and intestinal fluids to evaluate immunogen protection from degradation. In addition to appropriate pH's, pertinent enzymes are added to the simulated solutions (gastric: pepsin; intestinal: lipase, protease and pancreas) so that a step-wise assessment of various GI components can challenge the stability of the delivery particles. Antigen stability will be monitored by ELISA, Western Blot, SDS-PAGE, extrinsic fluorescence, and BCA assay. The delivery particles are also stored at 37° C and the stability of the antigen (SpeA/B) is monitored by ELISA, Western Blot, SDS-PAGE, extrinsic fluorescence, and BCA assay.

### Example 14

### Immune Cell Stimulation

Vaccine formulations from Example 12 exhibiting enhanced thermal stability and protection from gastric degradation are evaluated on their ability to stimulate immune cells to proliferate. Vaccines are combined with human PBMCs to determine whether the vaccine retains immune stimulating function following processing, storage, and exposure to simulated gastric fluid.

### Example 15

### Stability Study with CRM Oral Suspension

The solution and oral suspension formulations utilized for this stability study are presented in Table 8. As to the solution formulation 1.143 ml of 3.5 mg/ml CRM (in 20 mM Tris pH 7.5), 1.6 g of mannitol, 0.4 g of sucrose, and 18.8 ml of 20 mM Tris pH 7.5 were combined in a formulation vessel and mixed for 30 minutes. The solution was then sprayed into 20 ml of 150 mM sodium acetate solution at pH 4. The resulting solution was then divided into 1 ml aliquots and half were stored at 25° and the other half at 37° C.

As to the oral suspension formulation 1.143 ml of 3.5 mg/ml CRM (in 20 mM Tris pH 7.5), 1 ml of a 1% Eudragit solution (in 20 mM tris pH 7.5), 1.6 g of mannitol, 0.4 g of sucrose, and 17.8 ml of 20 mM Tris pH 7.5 were combined in a formulation vessel and mixed for 30 minutes. The solution was then sprayed into 20 ml of 150 mM sodium acetate solution at pH 4. The resulting suspension was then divided into 1 ml aliquots and half were stored at 25° and the other half at 37° C at pH 4.

**Table 8. Formulations used to evaluate the stability of CRM.**

| Group | Formulation |
|---|---|
| solution | 100 µg/ml CRM, 8% mannitol, 2% sucrose, 20 mM Tris |
| oral suspension | 100 µg/ml CRM, 0.05% Eudragit, 8% mannitol, 2% sucrose, 20 mM Tris |

Samples of both the solution and oral suspension were removed from 25° C storage at time 0, 1, 14, 28, 42, and 56 days and from 37° C storage at time 0, 1, 7, 14, 21, and 28 days. Following removal from storage samples were immediately frozen at -20° C. The epitope availability of CRM in each sample was then determined.

Epitope availability was determined by normalizing the response from an antigen specific ELISA assay to the total protein in the sample as determined by BCA assay. Prior to assay the oral suspension samples were dissolved by diluting the sample with 3 ml of 20 mM Tris at pH 9. Stock CRM stored at -20° C was used as the standard for the ELISA and BCA assay and was diluted to the appropriate concentrations in 20 mM Tris, 8% mannitol, and 2% sucrose. For the BCA assay, 25 µl of each standard and each sample was placed in triplicate on a 96 well plate. 200 ml of BCA reagent was then added to each well and the plate was incubated for 45 minutes at 37° C. After cooling the plate to room temperature the absorbance of each well was measured at 570 nm. The protein concentration was then determined by calculation from the standard curve.

For the ELISA, samples were diluted to 1 µg/ml CRM with 20 mM Tris pH 9 and 100 µl was placed in duplicate on a 96 well plate. Standards were also diluted in 20 mM Tris pH 9 and 100 µl was placed in duplicate on the 96 well plate. The plate was incubated for 1 hour at 37° C and then washed 3 times with 100 µl/well, 20 mM PO₄, 150 mM NaCl, and 0.05% Tween 20 washing buffer. Next, 100 µl/well BSA blocking buffer was added to plate and the plate was incubated at 37° C for 1 hour. The plate was washed 3 times with 100 µl/well washing buffer. Then 100 µl/well of a 1:250 dilution of anti-CRM-biotin antibody was added to each well and the plate was incubated for 1 hour at 37° C. The plate was washed again 3 times with 100 µl/well washing buffer. Next, 100 µl/well of a 1:3,000 dilution of streptavidin-HRP was added to each well and the plate was incubated for 1 hour at 37° C. The plate was washed 3 times with 100 µl/well washing buffer. Then 100 µl/well of TMB reagent was added to each well and the plate was incubated at room temperature for 20 minutes. After incubation100 µl/well of 3 M H₂SO₄ was added to the plate to stop the reaction. The absorbance was measured at 450 nm. The concentration of each sample was determined from the standard curve.

Epitope availability of each formulation was determined by dividing the protein concentration determined by BCA by the epitope concentration determined by ELISA to give the epitope availability. (Table 9) The data demonstrates that formulation as an oral suspension increases the stability of CRM. (Fig. 14) The first order degradation rate constants were determined for each of the formulations at both temperatures. (Table 10) These results demonstrate a 68% increase in stability when CRM is formulated as an oral suspension and stored at 25° C and a 54% increase in stability when stored at 37° C. When the data is plotted as the cumulative percent of antigen lost over time it can be also be seen how the oral suspension enhances stability of CRM. (Fig. 15) It takes less than 1 day for 50% of the epitopes in the solution formulation to be lost with storage at either 25° C or 37° C. However, for the oral suspension increases the time it takes for 50% loss to 12 days at 25° C and 7 days for 37° C. All of the data demonstrates a significant increase in the stability of CRM when formulated as an oral suspension verses a traditional liquid formulation.

**Table 9. The epitope availability was determined for each formulation. The formulations were stored at pH 4. Time points for 25° C were 0, 1, 14, 28, 42, and 56 days. Time points for 37° C were 0, 1, 7, 14, 21, and 28 days.**

| | Solution | | Oral Suspension | |
|---|---|---|---|---|
| Time Point | 25C | 37C | 25C | 37C |
| 0 | 0.576 | 0.576 | 0.576 | 0.576 |
| 1 | 0.213 | 0.213 | 0.548 | 0.548 |
| 2 | 0.085 | 0.043 | 0.247 | 0.139 |
| 3 | 0.053 | 0.032 | 0.233 | 0.068 |
| 4 | 0.037 | 0.019 | 0.166 | 0.064 |
| 5 | 0.019 | 0.018 | 0.105 | 0.055 |

**Table 10. First order rate constants calculated for the degradation of CRM at 25° C and 37° C.**

| | k (days-1) | |
|---|---|---|
| Temperature (°C) | Traditional Liquid | Oral Platform |
| 25 | 0.0406 | 0.0267 |
| 37 | 0.0715 | 0.0387 |

### Example 16

### Stability Study with CRM Dry Powder

The solution and dry powder formulations utilized for this stability study are presented in Table 11. For the solution formulation 1.143 ml of 3.5 mg/ml CRM (in 20 mM PO₄ pH 7.5), 1% Eudragit solution (in 20 mM PO₄ pH 7.5), 1.6 g of mannitol, 0.4 g of sucrose, and 17.8 ml of 20 mM PO₄ pH 7.5 were combined in a formulation vessel and mixed for 30 minutes. The resulting solution was then divided into 1 ml aliquots and half were stored at 25° and the other half at 37° C.

For the dry powder formulation 1.143 ml of 3.5 mg/ml CRM (in 20 mM Tris pH 7.5), 1 ml of a 1% Eudragit solution (in 20 mM PO₄ pH 7.5), 1.6 g of mannitol, 0.4 g of sucrose, and 17.8 ml of 20 mM PO₄ pH 7.5 were combined in a formulation vessel and mixed for 30 minutes. The solution was then sprayed into 20 ml of 150 mM sodium acetate solution at pH 4. The resulting suspension was then dried into a powder utilizing atmospheric spray freeze drying. The powder was divided into 100 mg samples and half were stored at 25° and the other half at 37° C.

**Table 11. Formulations used to evaluate the stability of CRM.**

| Group | Formulation |
|---|---|
| solution | 100 µg/ml CRM, 0.05% Eudragit, 8% mannitol, 2% sucrose, 20 mM PO4 |
| dry powder | 100 µg/ml CRM, 0.05% Eudragit, 8% mannitol, 2% sucrose, 20 mM PO4 |

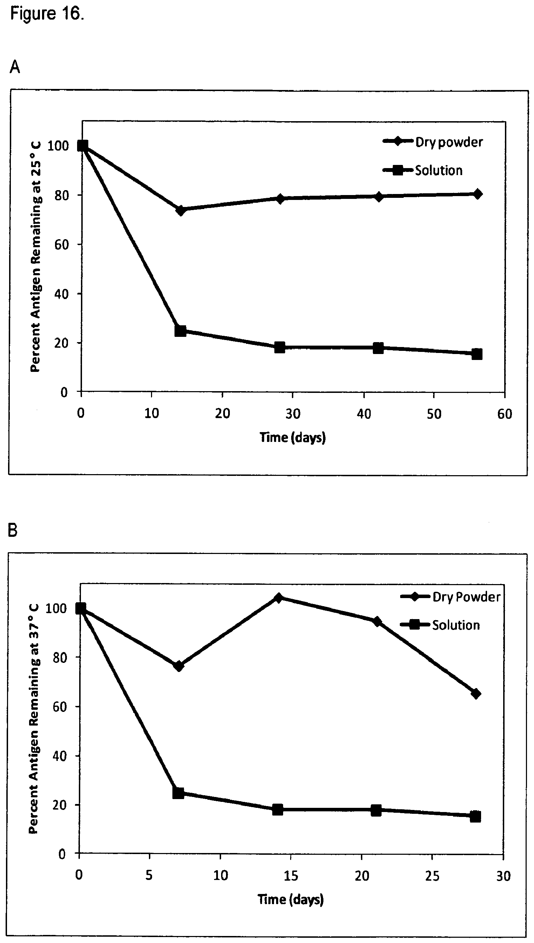

For the stability study samples were removed from 25° C storage at time 0, 1, 14, 28, 42, and 56 days and from 37° C storage at time 0, 1, 7, 14, 21, and 28 days. Following removal from storage samples were immediately frozen at - 20° C. Epitope availability of the CRM in each sample was determined as described in Example 15. While initially there was some loss in epitope availability in the dry powder following processing the remaining CRM epitopes were extremely stable even at 37° C. (Table 12)(Fig. 16) Formulation as a dry powder further enhances the stability of CRM.

**Table 12. The epitope availability was determined for each formulation. Time points for 25° C were 0, 1, 14, 28, 42, and 56 days. Time points for 37° C were 0, 1, 7, 14, 21, and 28 days.**

| | Solution | | Dry Powder | |
|---|---|---|---|---|
| Time Point | 25C | 37C | 25C | 37C |
| 0 | 0.696 | 0.696 | 0.303 | 0.413 |
| 1 | 0.173 | 0.173 | 0.224 | 0.317 |
| 2 | 0.129 | 0.129 | 0.239 | 0.432 |
| 3 | 0.127 | 0.127 | 0.242 | 0.393 |
| 4 | 0.110 | 0.110 | 0.246 | 0.272 |

Where ranges are given herein, the endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

## Claims

1. An immunological composition for oral administration comprising one or more C-type lectin (CTL) receptor ligands, one or more aluminum adjuvants and one or more antigens and one or more acrylic resin polymers.

2. The immunological composition of claim 1, wherein the acrylic resin polymer is a water-insoluble copolymer of ethyl acrylate (EA), methyl methacrylate (MM) and trimethylammoniumethyl methacrylate chloride.

3. The immunological composition of claim 1 further comprising a cryoprotectant, optionally wherein the cryoprotectant is selected from the group consisting of trehalose, mannitol, lactose, sorbitol, and sucrose, and combinations thereof.

4. A method of making an orally administerable immunogenic composition comprising: adsorbing an antigen and a C-type lectin (CTL) receptor ligand to an aluminum adjuvant; adding an acrylic resin polymer having pH dependent solubility to form a vaccine formulation; and adding the vaccine formulation to a low pH solution to precipitate the polymer thereby making an orally administerable immunogenic composition.

5. The method of claim 4 wherein the aluminum adjuvant is aluminum oxyhydroxide.

6. The method of claim 4 wherein the CTL-receptor ligand is a saccharide.

7. The method of claim 4, wherein the acrylic resin is a water-insoluble copolymer of ethyl acrylate (EA), methyl methacrylate (MM) and trimethylammoniumethyl methacrylate chloride.

8. The method of claim 4 further comprising the step of atmospheric spray freeze drying the orally administerable immunogenic composition.

## Patentansprüche

1. Immunologische Zusammensetzung zur oralen Verabreichung, die einen oder mehrere C-Typ-Lektin(CTL)-Rezeptorliganden, einen oder mehrere Aluminiumhilfsstoffe und einen oder mehrere Antigene und einen oder mehrere Acrylharzpolymere beinhaltet.

2. Immunologische Zusammensetzung gemäß Anspruch 1, wobei das Acrylharzpolymer ein wasserunlösliches Copolymer von Ethylacrylat (EA), Methylmethacrylat (MM) und Trimethylammoniumethylmonacrylatchlorid ist.

3. Immunologische Zusammensetzung gemäß Anspruch 1, die ferner ein Kryoprotektivum beinhaltet, wobei optional das Kryoprotektivum aus der Gruppe ausgewählt ist, die aus Trehalose, Mannitol, Laktose, Sorbitol und Saccharose und Kombinationen davon besteht.

4. Verfahren zum Herstellen einer oral verabreichbaren, immunogenen Zusammensetzung, das Folgendes beinhaltet: Absorbieren eines Antigens und eines C-Typ-Lektin(CTL)-Rezeptorliganden an einem Aluminiumhilfsstoff; Hinzufügen eines Acrylharzpolymers, das eine pH-abhängige Löslichkeit aufweist, um eine Vakzinformulierung zu bilden; und Hinzufügen der Vakzinformulierung zu einer Niedrig-pH-Lösung, um das Polymer zu präzipitieren, wodurch eine oral verabreichbare, immunogene Zusammensetzung hergestellt wird.

5. Verfahren gemäß Anspruch 4, wobei der Aluminiumhilfsstoff Aluminiumoxidhydroxid ist.

6. Verfahren gemäß Anspruch 4, wobei der CTL-Rezeptorligand ein Saccharid ist.

7. Verfahren gemäß Anspruch 4, wobei das Acrylharz ein wasserunlösliches Copolymer von Ethylacrylat (EA), Methylmethacrylat (MM) und Trimethylammoniumethylmonacrylatchlorid ist.

8. Verfahren gemäß Anspruch 4, das ferner den Schritt des atmosphärischen Sprühgefriertrocknens der oral verabreichbaren, immunogenen Zusammensetzung beinhaltet.

## Revendications

1. Composition immunologique pour administration par voie orale comprenant un ou plusieurs ligands du récepteur de la lectine de type C (CTL), un ou plusieurs adjuvants d'aluminium, un ou plusieurs antigènes et un ou plusieurs polymères de résine acrylique.

2. Composition immunologique selon la revendication 1, dans laquelle le polymère de résine acrylique est un copolymère insoluble dans l'eau d'acrylate d'éthyle (EA), de méthacrylate de méthyle (MM) et de chlorure de méthacrylate de triméthylammoniumméthyle.

3. Composition immunologique selon la revendication 1, comprenant en outre un cryoprotecteur, éventuellement dans laquelle le cryoprotecteur est choisi parmi le groupe constitué du tréhalose, du mannitol, du lactose, du sorbitol, du saccharose et de combinaisons des ceux-ci.

4. Procédé de fabrication d'une composition immunogène administrable par voie orale comprenant : l'adsorption d'un antigène et d'un ligand du récepteur de la lectine de type C (CTL) sur l'adjuvant d'aluminium ; l'ajout d'un polymère de résine acrylique ayant une solubilité dépendante du pH pour former une formulation de vaccin ; et l'ajout de la formulation de vaccin à une solution de bas pH pour précipiter le polymère, produisant ainsi une composition immunogène administrable par voie orale.

5. Procédé selon la revendication 4, dans lequel l'adjuvant d'aluminium est de l'oxyhydroxyde d'aluminium.

6. Procédé selon la revendication 4, dans lequel le ligand récepteur de la CTL est un saccharide.

7. Procédé selon la revendication 4, dans lequel la résine acrylique est un copolymère insoluble dans l'eau d'acrylate d'éthyle (EA), de méthacrylate de méthyle (MM) et de chlorure de méthacrylate de triméthylammoniuméthyle.

8. Procédé selon la revendication 4, comprenant en outre l'étape de lyophilisation par vaporisation atmosphérique de la composition immunogène administrable par voie orale.
